# FASCICULE DE BREVET EUROPEEN

(11) **EP 1 401 359 B1**
(45) Date de publication et mention de la délivrance du brevet: **26.08.2009**
(21) Numéro de dépôt: 02764940.9
(22) Date de dépôt: 04.07.2002
(51) Int. Cl.: A61F 2/24

(54) **ENSEMBLE PERMETTANT LA MISE EN PLACE D'UNE VALVE PROTHETIQUE DANS UN CONDUIT CORPOREL**
ANORDNUNG ZUM EINSETZEN EINER VENTILPROTHESE IN EIN KÖRPERGEFÄSS
KIT ENABLING A PROSTHETIC VALVE TO BE PLACED IN A BODY ENABLING A PROSTHETIC VALVE TO BE PUT INTO PLACE IN A DUCT IN THE BODY

(30) Priorité: 04.07.2001 FR 0108898
(43) Date de publication de la demande: 31.03.2004
(73) Titulaire: CoreValve, Inc., Irvine, CA 92618 (US)
(72) Inventeur: Séguin, Jacques, Windsor, Berks (GB)
(74) Mandataire: Jeannet, Olivier
(86) Numéro de dépôt international: PCT/FR2002/002352
(87) Numéro de publication internationale: WO 2003/003949

(56) Documents cités:
- EP-A- 1 057 460
- WO-A-98/29057
- DE-A- 19 857 887
- US-A- 3 657 744
- US-A- 5 851 232
- US-A- 5 957 949

## Description

La présente invention concerne un ensemble permettant la mise en place d'une valve prothétique dans un conduit corporel, notamment une valve cardiaque et en particulier une valve aortique.

Les documents WO 91/17720, WO 98/29057 et EP 1 057 460 décrivent chacun un tel ensemble, comprenant :
- la valve prothétique à implanter ;
- une armature radialement expansible, dite "stent", propre, à l'état expansé, à prendre appui contre la paroi du conduit corporel à équiper, cette prise d'appui permettant d'immobiliser ce stent par rapport à cette paroi ; et
- des moyens de fixation de la valve au stent.

La mise en place du stent permet ainsi le montage de la valve dans le conduit corporel, éliminant la nécessité d'un abord par l'extérieur et donc d'une intervention chirurgicale directe.

Cette technique toutefois a pour inconvénients essentiels d'induire un risque d'endommagement de la valve par le ballonnet utilisé pour réaliser l'expansion du stent, et de limiter la force d'expansion qu'il est possible d'imprimer au stent. Cette limitation a une répercussion sur l'ancrage du stent, rendant possible un déplacement dudit ensemble. Cette limitation a également une répercussion sur l'étanchéité du stent au niveau de l'anneau valvulaire, qui est particulièrement affectée lorsque des zones calcifiées confèrent à l'anneau valvulaire une forme irrégulière et/ou une certaine rigidité.

Un autre inconvénient de la technique antérieure est de lier directement les commissures des valvules au stent. Il en résulte qu'une expansion du stent, et donc de la valve, différente de celle prévue peut entraîner une mauvaise coaptation des valvules et donc un fonctionnement défectueux de la valve. Le stent doit par conséquent faire l'objet d'une expansion prédéterminée qui empêche, ou rend difficile, l'adaptation de ce stent à la variabilité anatomique.

La technique antérieure a également pour inconvénients, en cas d'implantation d'une valve aortique, de nécessiter un positionnement très exact du stent dans l'aorte afin que la valve se trouve placée en regard de l'anneau valvulaire natif, et d'induire un risque de bouchage des orifices des artères coronaires débouchant au niveau des ostia coronaires.

La présente invention vise à remédier à ces différents inconvénients.

L'ensemble qu'elle concerne comprend, de manière connue en soi :
- la valve prothétique à implanter ;
- une armature radialement expansible, dite "stent", comprenant au moins une zone destinée à être expansée pour permettre à ce stent, à l'état expansé, de prendre appui contre la paroi du conduit corporel à équiper, cette prise d'appui permettant d'immobiliser ce stent par rapport à cette paroi ; et
- des moyens de montage de la valve par rapport au stent, permettant de relier la valve au stent de telle sorte que la mise en place du stent permette le montage de la valve dans le conduit corporel,
des moyens d'expansion tel qu'un cathéter à ballonnet étant prévus pour provoquer l'expansion du stent au niveau du site d'implantation.

Selon l'invention, la valve et le stent sont conformés de telle sorte que la valve est située, au moment où est réalisée l'expansion du stent, en dehors de la ou des zones du stent soumises auxdits moyens d'expansion.

L'invention consiste ainsi à séparer la valve et cette ou ces zones à expanser, de sorte que l'expansion du stent peut être réalisée avec une force d'expansion adaptée à un parfait ancrage de ce stent dans la paroi du conduit corporel à équiper, et sans aucun risque de destruction ou d'altération de la valve.

Selon une possibilité, le stent comprend une zone de montage de la valve, qui est distincte de la ou des zones du stent à expanser, et lesdits moyens de montage relient la valve à cette zone de montage.

L'expansion du stent provoque alors le déploiement de la valve.

Selon une autre possibilité, lesdits moyens de montage sont conformés de telle sorte que la valve est mobile axialement par rapport au stent entre une position de non-implantation dans laquelle elle se trouve en dehors de la ou des zones du stent devant être expansées, et une position d'implantation, qu'elle peut atteindre après expansion du stent dans le conduit corporel, dans laquelle elle est immobilisée axialement par rapport au stent.

La valve peut alors former un sous-ensemble séparé du stent avant la mise en place de ce stent dans le conduit corporel, et peut être mise en place dans le stent une fois celui-ci implanté. Alternativement, la valve est reliée au stent avant la mise en place de ce stent dans le conduit corporel à traiter, et est par conséquent introduite dans ce conduit avec le stent ; lesdits moyens de montage comprennent alors des moyens de déplacement pour, une fois l'expansion du stent réalisée, déplacer la valve entre ladite position de non-implantation et ladite position d'implantation.

Lesdits moyens de montage peuvent alors comprendre une ou plusieurs des dispositions suivantes :
- des organes d'accrochage tels que des pointes, des crochets ou des griffes montés sur la valve, propres à être insérés dans la paroi délimitant ledit conduit corporel ; ces organes d'accrochage peuvent être orientés radialement par rapport à la valve, de manière à pouvoir être insérés dans ladite paroi lors du déploiement radial de la valve, ou peuvent être orientés tangentiellement par rapport à la valve, de manière à pouvoir être insérés dans ladite paroi lors d'un pivotement de la valve autour de son axe ou lors d'un mouvement longitudinal par rapport au stent ;
- des vésicules ruptibles remplies de colle biologique ou autre produit adhésif approprié, placées sur la face externe de la valve, ces vésicules étant propres à se rompre lorsque la valve est amenée dans sa position d'implantation, notamment par écrasement entre la valve et le stent ;
- au moins un bandeau ou un fil circulaire ou hélicoïdal intégré dans la paroi périphérique de la valve, présentant une mémoire de forme telle qu'il maintient la valve plaquée contre le stent dans la position d'implantation de cette valve ;
- des conduits aménagés dans, ou fixés sur, la paroi périphérique de la valve et des tiges aménagées sur le stent, ou inversement, ces tiges pouvant être engagées et pouvant coulisser au travers de ces conduits lors du passage de la valve de sa position de non-implantation à sa position d'implantation, des moyens tels que des crochets pouvant être prévus pour immobiliser ces conduits par rapport à ces tiges dans ladite position d'implantation ; des fils peuvent être reliés aux extrémités desdites tiges et peuvent passer au travers desdits conduits, pour guider facilement ces tiges dans ces conduits.

De préférence, les moyens de montage de la valve par rapport au stent sont conformés de manière à permettre, au-delà d'un seuil d'expansion du stent, une expansion différente de la valve et du stent, de sorte qu'une variation dans le degré d'expansion du stent n'a pas d'incidence sur le degré d'expansion de la valve.

La valve n'est ainsi pas reliée directement au stent, et n'est en particulier pas reliée au stent au niveau des commissures de ses valvules ; elle peut présenter, en position expansée du stent, un diamètre prédéterminé qui lui est propre, indépendant du diamètre du stent. Après implantation, la valve a donc une configuration permettant son bon fonctionnement quel que soit le degré d'expansion du stent, et cette expansion du stent peut être adaptée à la variabilité anatomique rencontrée au niveau du site d'implantation.

Le stent et/ou la valve peuvent comprendre un ou plusieurs éléments limitant le diamètre maximal d'expansion de la valve, notamment au niveau des points commissuraux de cette valve. Ces éléments peuvent être des fils longitudinaux que comprend le stent ou un élément d'armature que comprend la valve.

De préférence, la valve présente une paroi périphérique d'un diamètre non constant dans le sens axial, notamment une forme tronconique dont le diamètre se réduit dans la direction distale, et la zone du stent destinée à recevoir cette paroi périphérique de la valve présente une forme correspondant à celle de cette paroi périphérique.

Cette paroi périphérique et cette zone du stent définissent ainsi une position déterminée de montage de la valve dans le stent, et assurent le maintien en position de la valve dans le stent.

Le stent présente avantageusement une portion médiane de diamètre plus réduit que ses portions d'extrémité. Il peut notamment présenter une forme générale en deux troncs de cône inversés ou en diabolo.

Dans le cas ou l'ensemble selon l'invention permet le montage d'une valve aortique, le stent présente ainsi un éloignement de la paroi du conduit corporel, notamment par une forme conique ou en diabolo, permettant le passage du fluide corporel vers les coronaires au niveau des ostia coronaires. La valve présente une forme correspondant à la zone du stent au niveau de laquelle elle est destinée à être montée.

Avantageusement,
- la valve présente une paroi périphérique ;
- le stent présente, dans le prolongement, du côté distal, de la zone du stent destinée à recevoir la valve, une portion rabattable ; cette portion rabattable est mobile entre une position étendue, dans laquelle elle se trouve dans le prolongement de ladite zone du côté distal, et une position rabattue, dans laquelle elle est placée contre la face interne de la paroi périphérique de la valve et emprisonne cette paroi périphérique entre elle et ladite zone du stent, et
- des moyens de maintien sont prévus pour maintenir cette portion rabattable dans cette position rabattue.

La paroi périphérique de la valve est ainsi plaquée contre le stent, ce qui assure l'étanchéité de la valve par rapport au stent.

Selon une forme de réalisation préférée de l'invention dans ce cas, lesdits moyens de maintien sont constitués par un fil en un matériau rigide mais présentant un degré de souplesse élastique, tel qu'un matériau métallique, ayant une forme ondulée et s'étendant sur l'ensemble de la circonférence de ladite portion rabattable.

De préférence, le stent comprend une gaine en un matériau biocompatible imperméable, le recouvrant au moins partiellement.

Cette gaine forme à la fois une base de fixation pour la valve et un moyen d'étanchéité entre le stent et la paroi du conduit corporel.

La gaine peut avantageusement présenter des ouvertures latérales, propres à venir en regard des ostia coronaires au moment de l'implantation et permettant ainsi d'éviter toute zone de stagnation ou de non-circulation du sang.

Avantageusement, dans le cas où l'ensemble selon l'invention comprend ladite portion rabattable, cette portion rabattable est constituée par un prolongement de ladite gaine, qui forme un manchon au-delà de ladite zone du stent destinée à recevoir la valve.

Une parfaite étanchéité est ainsi obtenue entre la valve et le stent.

De préférence, le stent présente, fixée sur ladite gaine, au moins une chambre périphérique gonflable, pouvant être gonflée de manière à former un joint assurant l'étanchéité entre le stent et la paroi du conduit corporel à équiper.

Cette étanchéité est ainsi assurée nonobstant la présence éventuelle de portions calcifiées conférant à un anneau cardiaque une forme irrégulière.

Avantageusement dans ce cas, le stent présente deux chambres périphériques gonflables placées de part et d'autre de la portion du stent destinée à prendre appui contre un anneau valvulaire cardiaque.

Le stent peut présenter une portion cylindrique, propre à prendre appui contre un anneau valvulaire cardiaque, et une portion distale, reliée à cette portion cylindrique.

Cette portion distale forme au moins en partie ladite zone destinée à recevoir la paroi périphérique de la valve. Elle a pour avantage que ladite paroi en matériau biocompatible imperméable se trouve, au niveau de cette portion, à distance de la paroi du conduit corporel, ce qui, dans le cas d'implantation d'une valve aortique, permet d'éliminer le risque de masquer les ostia coronaires.

Le stent peut également présenter une portion proximale tronconique ou évasée, dont le diamètre va en se réduisant dans la direction distale, propre, dans le cas de l'implantation d'une valve cardiaque, à prendre appui contre la paroi du ventricule ou de l'oreillette correspondante du coeur.

Cette portion proximale permet de définir la position du stent, et donc ultérieurement de la valve, par rapport à la zone d'implantation. Elle contribue également à assurer une parfaite immobilisation du stent.

Le stent peut également présenter une portion supplémentaire d'appui reliée par des tiges filiformes à ladite portion distale ou à ladite portion cylindrique, ces tiges filiformes ayant des longueurs telles que cette portion supplémentaire d'appui est platée au-delà des ostia coronaires.

Selon une caractéristique supplémentaire, le stent présente des crochets mobiles entre une position de retrait, qu'ils occupent avant expansion du stent, et une position de déploiement, dans laquelle ils sont amenés lors du déploiement du stent et dans laquelle ils sont insérés dans une paroi délimitant le conduit corporel.

Le stent peut en outre présenter une portion proche de l'anneau valvulaire, ou en regard ou sur cet anneau valvulaire, ayant une force radiale élevée, c'est-à-dire une force radiale permettant de gommer les irrégularités anatomiques locales, comme par exemple des calcifications, et ce dans le but de renforcer l'étanchéité de la jonction entre le stent, la gaine et la paroi du conduit traité.

Cette portion peut être deployée à l'aide d'un système d'expansion à force radiale élevée et peu compliant, par exemple un ballonnet.

Pour sa bonne compréhension, l'invention est à nouveau décrite ci-dessous en référence au dessin schématique annexé représentant, à titre d'exemples non limitatifs, trois formes de réalisation possibles de l'ensemble qu'elle concerne.
La figure 1 est une vue de côté d'une armature expansible, dite "stent" qu'il comprend ;
la figure 2 est une vue en coupe longitudinale d'une gaine qu'il comprend, selon une première forme de réalisation ;
la figure 3 est une vue en coupe longitudinale d'une valve cardiaque qu'il comprend, selon cette première forme de réalisation ;
la figure 4 est une vue d'un détail du stent, à échelle agrandie ;
la figure 5 est une vue d'un autre détail du stent, à échelle agrandie, dans un état de non-expansion du stent ;
la figure 6 est une vue du même détail, en coupe selon la ligne VI-VI de la figure 5 ;
la figure 7 est une vue similaire à la figure 5, dans un état d'expansion du stent ;
la figure 8 et une vue du même détail, en coupe selon la ligne VIII-VIII de la figure 7 ;
la figure 9 est une vue en coupe longitudinale de l'ensemble selon l'invention, après implantation dans une aorte ;
la figure 10 est une vue en coupe longitudinale de la gaine et de la valve que comprend l'ensemble selon l'invention, selon une deuxième forme de réalisation, avec le cathéter permettant d'amener la valve dans cette gaine ;
la figure 11 est une vue en perspective de la valve selon la deuxième forme de réalisation ;
la figure 12 est une vue similaire à la figure 10, après mise en place de la valve ;
la figure 13 est une vue similaire à la figure 9, de l'ensemble selon une troisième forme de réalisation ;
la figure 14 est une vue en perspective de la valve susceptible d'être mise en place dans le stent montré sur la figure 13, et
les figures 15 à 17 sont des vues de l'ensemble selon la troisième forme de réalisation, en coupe selon respectivement les lignes XV-XV, XVI-XVI et XVII-XVII de la figure 13.

Dans ces trois formes de réalisation, les éléments ou parties identiques ou similaires, qui se retrouvent d'une forme de réalisation à l'autre, sont désignés par les mêmes références numériques.

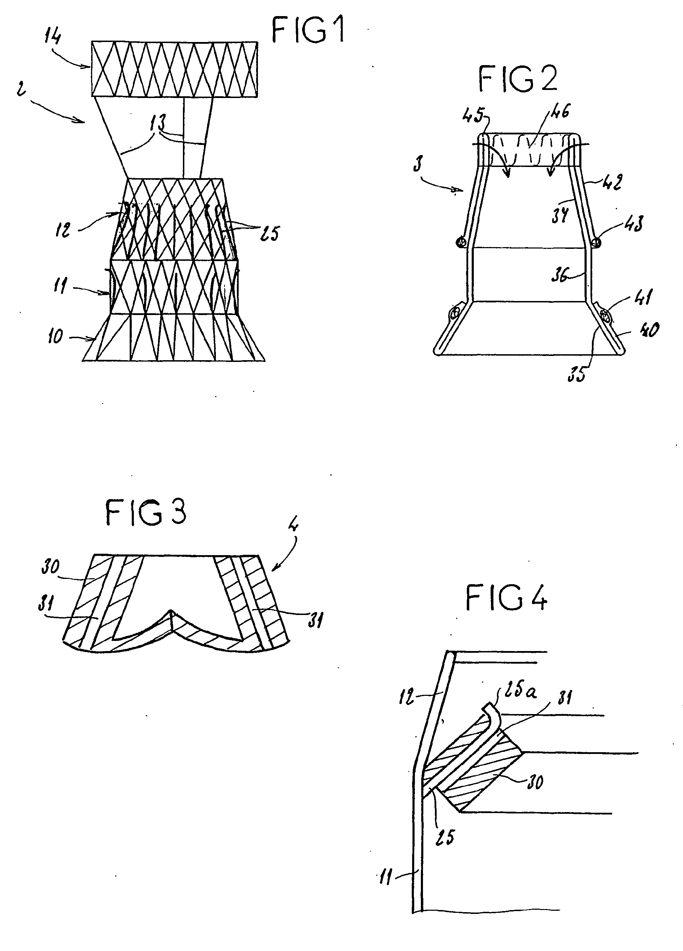

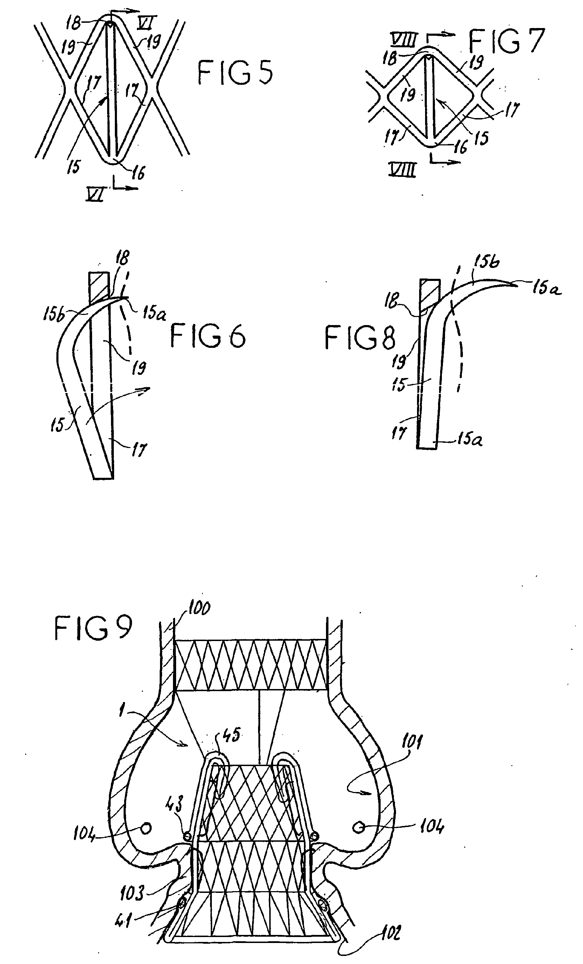

Les figures 1 à 3 représentent respectivement une armature expansible 2, dite "stent", une gaine 3 et une valve prothétique 4. Ce stent 2, cette gaine 3 et cette valve 4 composent un ensemble 1, visible sur la figure 9, permettant la mise en place de la valve 4 dans une aorte 100, montrant l'emplacement des ostia coronaires 101 et la naissance des coronaires 104.

En référence à la figure 1, il apparaît que le stent 2 comprend successivement, d'une extrémité axiale à l'autre, dans le sens proximal / distal, une portion proximale tronconique 10, une portion cylindrique proximale 11, une portion tronconique distale 12, plusieurs tiges de liaison 13 et une portion cylindrique distale 14.

Ce stent 2 est en matériau métallique, en acier ou en alliage à mémoire de forme. Ce matériau à mémoire de forme peut notamment être celui connu sous la dénomination "NITINOL".

Les portions 10 à 12 et 14 sont constituées d'un réseau de filaments formant des mailles juxtaposées en forme de losange ou, pour la portion 10, en forme de triangle. Le matériau constituant le stent 2 est tel que ces mailles peuvent passer d'une configuration contractée, dans laquelle les filaments sont proches les uns des autres, conférant aux mailles une forme allongée, à une configuration expansée, visible sur !a figure 1 et sur la figure 7 en détail, dans laquelle les filaments sont écartés les uns des autres.

Dans la configuration contractée, l'ensemble 1 peut être introduit dans l'aorte 100 au moyen d'un cathéter, jusqu'à la zone au niveau de laquelle la valve prothétique 4 doit être implantée ; dans la configuration expansée, le stent 2 prend appui contre l'aorte 100, la paroi 102 du ventricule et l'anneau valvulaire natif 103 de la manière montrée par la figure 9, de telle sorte qu'il permet l'implantation de la valve 4 en lieu et place de la valve native, cette dernière ayant préalablement été supprimée si nécessaire.

En référence aux figures 1 et 9, il apparaît que la portion 10 a un diamètre allant en se réduisant dans la direction distale, cette portion 10 étant conformée pour, à l'état expansé, prendre appui contre la paroi 102 du ventricule du coeur.

La portion 11 présente, à l'état expansé, un diamètre tel qu'elle est à même de prendre appui contre l'anneau valvulaire natif 103 et une force radiale telle qu'elle peut repousser la valve native (ou ses résidus après exérèse partielle) contre l'anneau 103 de façon à assurer l'étanchéité à cet endroit. Cette portion 11 présente des crochets 15 déployables, visibles plus particulièrement sur les figures 5 à 8. Ces crochets 15 permettent l'ancrage du stent 2 à l'aorte 100 au travers de la gaine 3.

Comme le montrent les figures 5 et 6, chaque crochet 15 s'étend dans le sens longitudinal d'une maille, en étant relié de manière pivotante à une zone proximale 16 de raccordement de deux filaments proximaux 17. Ce crochet 15 présente une extrémité libre 15a recourbée et effilée, et une face 15b tournée vers l'intérieur du stent 2, de forme arrondie. La zone distale 18 de raccordement des deux autres filaments 19 située du côté opposé à la base du crochet 15 est à même de venir porter, au cours de l'expansion du stent 2, sur cette face 15b, ainsi que cela se déduit de la comparaison des figures 6 et 8. L'appui de cette zone 18 le long de cette face 15b permet de déployer le crochet 15 radialement vers l'extérieur du stent 2 et de maintenir ce crochet 15 en position déployée, dans laquelle son extrémité effilée 15a est insérée dans la paroi de l'anneau 103. Les crochets peuvent avoir une forme en hameçon afin d'empêcher leur retrait.

La portion 12 est directement reliée à la portion 11 et présente un diamètre allant en se réduisant dans la direction distale. Cette portion 12 est destinée à s'étendre au niveau des ostia coronaires 101 et à recevoir la valve 4. Sa forme tronconique permet de maintenir !a gaine 3 à distance des ostia coronaires 101 et donc de prévenir tout risque de recouvrement des orifices 104 des coronaires qui débouchent dans ceux-ci.

La portion 12 comprend en outre une série de bras internes 25 visibles plus particulièrement sur la figure 4. Chaque bras 25 est relié par son extrémité proximale à une zone de jonction 16 de deux filaments proximaux 17 d'une maille, à proximité de la portion 11, et présente une extrémité distale recourbée 25a. Ces bras 25 sont inclinés vers l'intérieur de la portion 12 avant mise en place de la valve 4 sur le stent 2, et la figure 4 montre que, dans cette position, ils peuvent recevoir la valve 4. Celle-ci comprend en effet une paroi périphérique 30 dans laquelle sont aménagés des tunnels longitudinaux 31 propres à recevoir les bras 25 ; ces derniers peuvent ensuite être rabattus contre la paroi de la portion 12, soit par déformation du matériau constituant les bras 25 et/ou la portion 12, soit par mémoire de forme lorsqu'un matériau à mémoire de forme est utilisé. Ces bras 25 permettent ainsi de monter la valve 4 dans la portion 12, comme montrée sur la figure 9.

Les tiges de liaison 13 relient le bord distal de la portion 12 au bord proximal de la portion 14. Elles sont régulièrement disposées sur la périphérie du stent 2 et, comme le montre la figure 9, ont une longueur suffisante pour que la portion 14 soit placée, après implantation, au-delà des ostia coronaires 101.

L'écartement de ces tiges 13 peut être bridé par un élément annulaire permettant de limiter le diamètre supérieur de la valve 4 à une taille prédéfinie.

La portion 14 présente quant à elle, à l'état expansé, un diamètre légèrement supérieur au diamètre interne de l'aorte 100, et vient en appui contre la paroi de cette dernière une fois le stent 2 mis en place. Cette portion 14 peut être équipée de crochets 15.

La gaine 3 est en un matériau biocompatible imperméable, tel que du tissu péricardique, du matériau connu sous la dénomination "DACRON" ou un polymère, tel que le polyuréthane, et présente des portions 35, 36 et 37. Ces portions 35, 36 et 37 sont propres à être reliées respectivement aux portions 10, 11 et 12, et à épouser étroitement ces portions 10 à 12, à l'état expansé de celles-ci. La liaison entre la gaine 3 et les portions 10 à 12 est réalisée, au moment de !'assemb!age de l'ensemble 1, par des coutures. La liaison peut également être réalisée par moulage d'un matériau polymérique.

Du côté proximal, la gaine 3 présente un rabat 40 s'étendant sur la face extérieure de la portion 35. Ce rabat 40 comporte, à proximité de son bord libre, une chambre périphérique gonflable 41. Cette chambre 41 peut être gonflée de manière à former un joint assurant une étanchéité entre la gaine 3 et la paroi du ventricule 102, sur le côté proximal de l'anneau valvulaire natif 103.

Du côté distal, la gaine 3 présente un rabat 42 s'étendant sur la face extérieure de la portion 12. Ce rabat 42 comporte, à proximité de son bord libre, une chambre périphérique gonflable 43, similaire à la chambre 41 et pouvant être gonflée de la même manière que cette dernière. Cette chambre 43 assure une étanchéité entre la gaine 3 et l'anneau 103, du côté distal de celui-ci.

Il apparaît sur la figure 2 que le rabat 42 forme un manchon distal 45, s'étendant au-delà du bord distal de la portion 12, et sur la figure 9 que ce manchon 45 est rabattable à l'intérieur de la portion 12. Ce manchon 45 inclut un fil métallique 46 s'étendant sur l'ensemble de sa circonférence, ce fil 46 ayant une forme ondulée et étant élastiquement déformable. La déformabilité de ce fil 46 permet au manchon 45 de passer de sa position étendue montrée sur la figure 2 à sa position rabattue montrée sur la figure 9, dans laquelle il est maintenu par rappel élastique du fil 46. Dans cette position rabattue, le manchon 45 est placé contre la face interne de la paroi périphérique 30 de la valve 4 et emprisonne cette paroi 30 entre lui et ladite portion 12.

La valve 4 peut être fabriquée en un matériau biologique ou en un matériau synthétique ou en une combinaison de ces deux types de matériaux. Sa paroi périphérique 30 présente une forme tronconique adaptée à son emboîtement étroit dans la portion 12 lorsque les bras 25 sont rabattus contre cette portion 12, ce qui assure une parfaite immobilisation de la valve 4 dans le stent 2.

L'assemblage de l'ensemble 1 est réalisé par mise en place de la gaine 3 sur le stent 2 et de la valve 4 sur les bras 25, le stent 2 étant à l'état contracté. L'ensemble 1 est ensuite placé dans un cathéter permettant son introduction dans le corps du patient, ce cathéter incluant un ou plusieurs ballonnets gonflables propres à déployer les portions 10, 11 et 14. Puis, ce cathéter est amené en position dans l'aorte 100. Les baronnets sont alors gonflés pour déployer les portions 10, 11 et 14 ; le déploiement forcé de la portion 11, qu'ils réalisent, permet d'assurer le déploiement des crochets 15 et de provoquer le déploiement de la portion 12, donc de la valve 4. Les chambres 41, 43 sont ensuite gonflées pour assurer l'étanchéité de la gaine 3 par rapport à l'anneau 103, et le manchon 45 est rabattu à l'intérieur de la portion 12 pour serrer la paroi périphérique 30 de la valve 4 contre cette portion 12.

Ainsi que cela apparaît de ce qui précède, dans l'ensemble 1, la valve 4 et le stent 2 sont conformés de telle sorte que la valve 4 est située en dehors de la ou des zones 10, 11, 14 devant être expansées. L'expansion du stent 2 peut être réalisée avec une force d'expansion adaptée à un parfait ancrage de ce stent 2 dans les parois réceptrices 100, 102, 103, et sans aucun risque pour la valve 4.

Les crochets 15 permettent d'assurer une parfaite immobilisation de l'ensemble 1 dans l'aorte 100, et les chambres 41, 43, ainsi que le manchon 45, permettent d'assurer une parfaite étanchéité de l'ensemble 1 par rapport à l'aorte 100.

Dans la deuxième forme de réalisation de l'ensemble 1, la valve 4 n'est pas pré-montée à l'intérieur du stent 2 mais est mise en place une fois l'expansion du stent 2 réalisée.

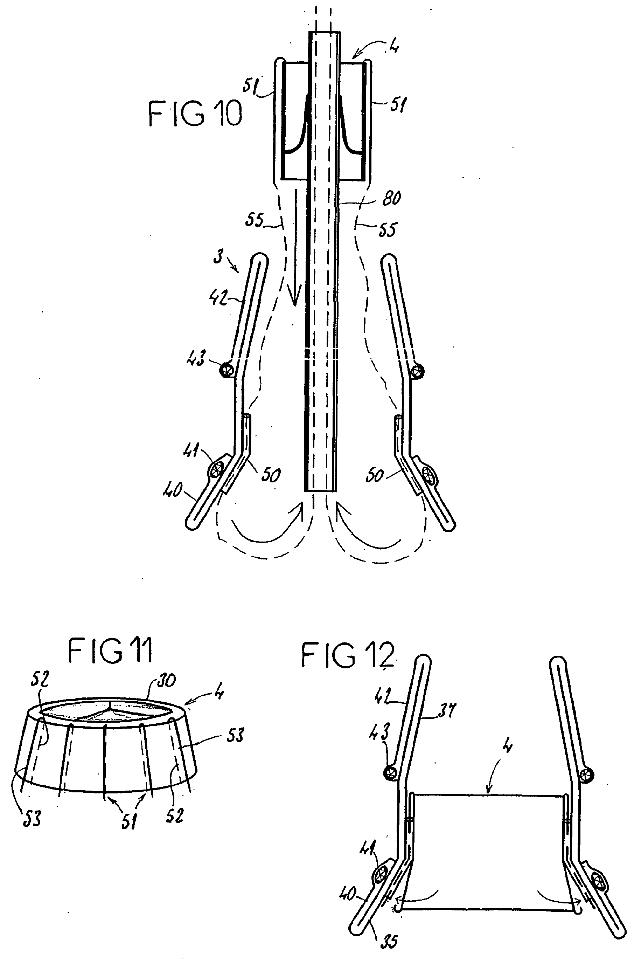

Comme le montre la figure 10, la gaine 3 présente alors des tubes internes 50 aménagés de manière à faire saillie à l'intérieur du stent 2 une fois cette gaine 3 engagée sur le stent 2. Ces tubes 50 peuvent notamment être cousus ou fixés par tout autre moyen à la gaine 3 après mise en place de celle-ci sur le stent 2.

En référence à la figure 11, il apparaît que la paroi périphérique 30 de la valve 4 présente, en lieu et place des tunnels 31, une série d'agrafes 51 en épingle. Chaque agrafe 51 présente un bras interne 52 engagé longitudinalement dans la paroi 30, et un bras externe 53 rectiligne, s'étendant le long de la face externe de la paroi 30. Les bras 53 se terminent par des extrémités recourbées et sont reliés à des fils 55 engagés et pouvant coulisser dans les tubes 50.

Comme le montre la figure 10, un cathéter 80 est utilisé pour amener la valve 4 en position dans la gaine 3. La valve 4 est engagée à coulissement sur le cathéter 80, et les fils 55 sont, après avoir traversé les tubes 50, engagés dans le cathéter 80 à partir de l'ouverture distale de celui-ci et traversent ce cathéter 80 afin d'être actionnés de l'extérieur. La mise en place et le déploiement de la valve 4 est réalisé en opérant une traction sur les différents fils 55 de manière à engager les différents bras 53 dans les tubes 50 respectifs. Les bras internes 52 peuvent comprendre (cf. figure 12) des crochets proximaux qui permettent de compléter le déploiement de la valve 4 en étant accrochés à la paroi de la gaine 3, par exemple au moyen du gonflement d'un ballonnet.

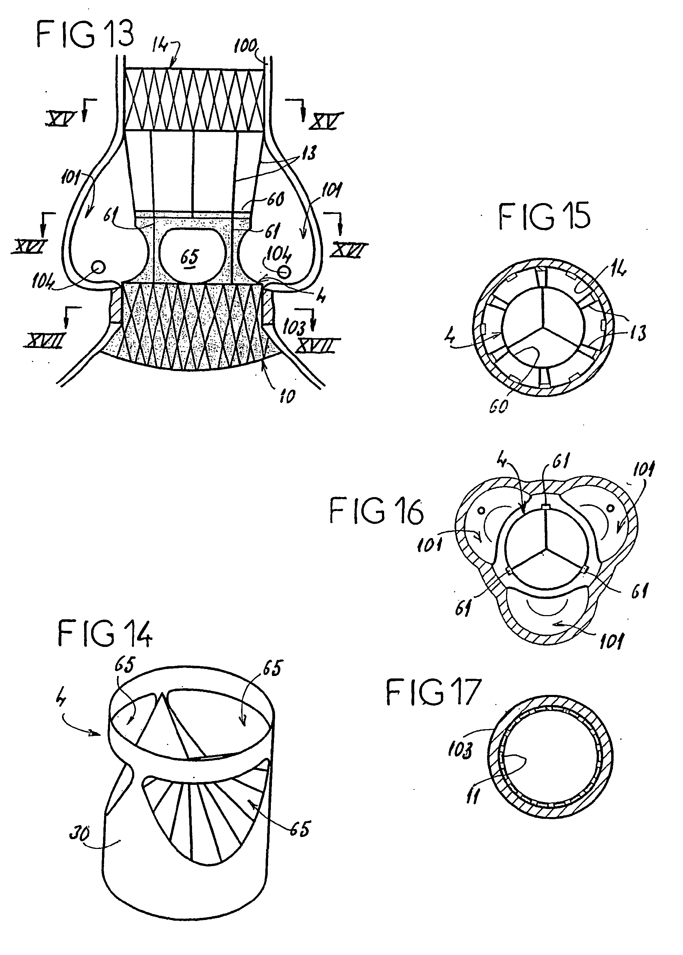

Dans la troisième forme de réalisation de l'ensemble 1 montrée par la figure 13, le stent 2 forme une cage médiane délimitée par un anneau 60 et par des fils longitudinaux 61, dans laquelle s'insère étroitement la valve 4. Celle-ci présente, ainsi que le montre la figure 14, une paroi latérale 30 de forme tubulaire dans laquelle sont aménagées trois ouvertures latérales 65. Ces ouvertures 65 se positionnent en regard des ostia coronaires 101 et permettent un écoulement naturel du sang, sans risque de stagnation au niveau de ces ostia coronaires 101.

L'invention fournit un ensemble 1 de mise en place d'une valve 4 dans un conduit corporel 100 présentant, par rapport aux ensembles homologues de la technique antérieure, les avantages suivants :
- élimination du risque d'endommagement de la valve 4 par le ou les ballonnets utilisés pour réaliser l'expansion du stent 2 ;
- possibilité d'imprimer une force d'expansion importante au stent 2, qui assure l'ancrage de l'ensemble 1 ; cette force d'expansion importante permet en outre d'assurer parfaitement cet ancrage grâce aux crochets 15 déployables ;
- élimination du risque de dilatation de la valve 4 au-delà d'un diamètre ne permettant plus son fonctionnement optimal, en particulier par perte de coaptation des valvules ;
- possibilité d'obtention d'une parfaite étanchéité de l'ensemble 1 au niveau de l'anneau valvulaire 103 et de la valve 4 ;
- élimination du risque de bouchage des ostia coronaires 101 ;
- maintien d'un écoulement du fluide corporel tout autour dudit ensemble 1 une fois celui-ci implanté.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Ensemble permettant la mise en place d'une valve prothétique (4) dans un conduit corporel, notamment une valve cardiaque et en particulier une valve aortique, comprenant :
- la valve prothétique (4) à implanter ;
- une armature (2) radialement expansible, dite "stent"; comprenant au moins une zone (10, 11, 14) destinée à être expansée pour permettre à ce stent (2), à l'état expansé, de prendre appui contre la paroi du conduit corporel (100) à équiper, cette prise d'appui permettant d'immobiliser ce stent (2) par rapport à cette paroi ; et '
- des moyens (25, 31 ; 50, 51) de montage de la valve (4) par rapport au stent (2), permettant de relier la valve (4) au stent (2) de telle sorte que la mise en place du stent (2) permette le montage de la valve (4) dans le conduit corporel (100),
des moyens d'expansion tel qu'un cathéter à ballonnet étant prévus pour provoquer l'expansion du stent (2) au niveau du site d'implantation ;
ensemble (1) **caractérisé en ce que** :
- l'armature (2) est unique, la partie de cette armature comprenant ladite au moins une zone (10, 11, 14) destinée à être expansée formant une pièce unitaire avec la partie de cette armature recevant la valve ou destinée à recevoir la valve, et
- la valve (4) et le stent (2) sont conformés de telle sorte que la valve (4) est située, au moment où est réalisée l'expansion du stent (2), en dehors de la ou des zones (10, 11, 14) du stent (2) soumises auxdits moyens d'expansion.

2. Ensemble (1) selon la revendication 1, **caractérisé en ce que** le stent (2) comprend une zone (12) de montage de la valve (4), qui est distincte de la ou des zones (10, 11, 14) du stent (2) à expanser, et **en ce que** lesdits moyens de montage (25, 31) relient la valve (4) à cette zone de montage (12).

3. Ensemble (1) selon la revendication 2, **caractérisé en ce que** lesdits moyens de montage (25, 31) comprennent des conduits (31) aménagés dans, ou fixés sur, la paroi périphérique (30) de la valve (4) et des bras (25) aménagées sur le stent (2), ou inversement, la paroi périphérique (30) de la valve (4) pouvant être engagée et pouvant coulisser sur ces bras (25).

4. Ensemble (1) selon la revendication 1, **caractérisé en ce que** lesdits moyens de montage (50, 51) sont conformés de telle sorte que la valve (4) est mobile axialement par rapport au stent (2) entre une position de non-implantation, dans laquelle elle se trouve en dehors de la ou des zones (10, 11, 14) du stent (2) devant être expansées, et une position d'implantation, qu'elle peut atteindre après expansion du stent (2) dans le conduit corporel (100), .dans laquelle elle est immobilisée axialement par rapport au stent (2):

5. Ensemble (1) selon la revendication 4, **caractérisé en ce que** lesdits moyens de montage (50, 51) comprennent une ou plusieurs des dispositions suivantes :
- des organes d'accrochage tels que des pointes, des crochets ou des griffes montés sur la valve, propres à être insérés dans la paroi délimitant ledit conduit corporel ; ces organes d'accrochage aptes à être orientés radialement par rapport à la valve, de manière à être aptes à être insérés dans ladite paroi lors du déploiement radial de la valve, ou sont aptes à être orientés tangentiellement par rapport à la valve, de manière à être aptes à être insérés dans ladite paroi lors d'un pivotement de la valve autour de son axe ou lors d'un mouvement longitudinal par rapport au stent ;
- des vésicules ruptibles remplies de colle biologique ou autre produit adhésif approprié, placées sur la face externe de la valve, ces vésicules étant propres à se rompre lorsque la valve est amenée dans sa position d'implantation, notamment par écrasement entre la valve et le stent ;
- au moins un bandeau ou un fil circulaire ou hélicoïdal intégré dans la paroi périphérique de la valve, présentant une mémoire de forme telle qu'il maintient la valve plaquée contre le stent dans la position d'implantation de cette valve ;
- des conduits (50) aménagés dans, ou fixés sur, la paroi périphérique de la valve et des tiges aménagées sur le stent, ou inversement, ces tiges (53.) étants aptes à être engagées et à coulisser au travers de ces conduits (50) lors du passage de la valve (4) de sa position de non-implantation à sa position d'implantation ;
- des moyens tels que des crochets sont aptes à immobiliser lesdits conduits (50) par rapport à ces tiges (53) dans ladite position d'implantation ;
- des fils (55) sont aptes à être reliés aux extrémités desdites tiges (53) et à passer au travers desdits conduits (50), pour guider facilement ces tiges (53) dans ces conduits (50).

6. Ensemble (1) selon la revendication 1, **caractérisé en ce que** les moyens (60) de montage de la valve (4) par rapport au stent (2) sont conformés de manière à permettre, au-delà d'un seuil d'expansion du stent, une expansion différente de la valve (4) et du stent (2), de sorte qu'une variation dans le degré d'expansion du stent (2) n'a pas d'incidence sur le degré d'expansion de la valve (4).

7. Ensemble (1) selon l'une des revendications 1 à 6, **caractérisé en ce que** le stent (2) et/ou la valve (4) comprennent un ou plusieurs éléments (60) limitant le diamètre final de la valve (4) notamment au niveau des points commissuraux des valvules de celle-ci.

8. Ensemble (1) selon l'une des revendications 1 à 7, **caractérisé en ce que** la valve (4) présente une paroi périphérique (30) d'un diamètre non constant dans le sens axial, notamment une forme tronconique dont le diamètre se réduit dans la direction distale, et **en ce que** la zone (12) du stent (2) destinée à recevoir cette paroi périphérique (30) de la valve (4) présente une forme correspondant à celle de cette paroi périphérique (30).

9. Ensemble (1) selon l'une des revendications 1 à 8, **caractérisé en ce que** le stent (2) présente une portion médiane de diamètre plus réduit que ses portions d'extrémité, notamment en deux troncs de cône inversés ou en diabolo, la valve présentant une forme correspondant à la zone du stent au niveau de laquelle elle est destinée à être montée.

10. Ensemble (1) selon l'une des revendications 1 à 9, **caractérisé en ce que** :
- la valve (4) présente une paroi périphérique (30) ;
- le stent (2) présente, dans le prolongement, du côté distal, de la zone (12) du stent (2) destinée à recevoir la valve (4), une portion rabattable (45) ; cette portion rabattable (45) est mobile entre une position étendue, dans laquelle elle se _ trouve dans le prolongement de ladite zone (12) du côté distal, et une position rabattue, dans laquelle elle est placée contre la face interne de la paroi périphérique (30) de la valve (4) et emprisonne cette paroi périphérique (30) entre elle et ladite zone (12) du stent (2), et
- des moyens de maintien (46) sont prévus pour maintenir cette portion rabattable (45) dans cette position rabattue.

11. Ensemble. (1) selon la revendication 10, **caractérisé en ce que** lesdits moyens de maintien sont constitués par un fil (46) en un matériau rigide mais présentant un degré de souplesse élastique, tel qu'un matériau métallique, ayant une forme ondulée et s'étendant sur l'ensemble de la circonférence de ladite portion rabattable (45).

12. Ensemble (1) selon l'une des revendications 1 à 11, **caractérisé en ce que** le stent (2) comprend une gaine (3) en un matériau biocompatible imperméable, le recouvrant au moins partiellement.

13. Ensemble (1) selon la revendication 12, pour la mise en place d'une valve aortique, **caractérisé en ce que** la gaine présente des ouvertures latérales (61), propres à venir en regard des ostia coronaires (101) au moment de t'implantation.

14. Ensemble (1) selon la revendication 12 ou la revendication 13, **caractérisé en ce que**, dans le cas où il comprend ladite portion rabattable (45), cette portion rabattable (45) est constituée par un prolongement de la gaine (3), qui forme un manchon au-delà de ladite zone (12) du stent (2) destinée à recevoir la valve (4).

15. Ensemble (1) selon l'une des revendications 12 à .14, **caractérisé en ce que** le stent (2) présente, fixée sur ladite gaine (3), au moins une chambre périphérique gonflable (41, 43), pouvant être gonflée de manière à former un joint assurant l'étanchéité entre le stent (2) et la paroi du conduit corporel (100) à équiper.

16. Ensemble (1) selon la revendication 15, **caractérisé en ce que** le stent (2) présente deux chambres périphériques gonflables (41, 43) placées de part et d'autre de la portion (11) du stent (2) destinée à prendre appui contre un anneau valvulaire cardiaque (103).

17. Ensemble (1) selon l'une des revendications 1 à 16, **caractérisé en ce que** le stent (2) présente une portion cylindrique (11), propre à prendre appui contre un anneau valvulaire cardiaque (103), et une portion distale (12), reliée à cette portion cylindrique (11).

18. Ensemble (1) selon l'une des revendications 1 à 17, **caractérisé en ce que** le stent (2) présente une portion proximale (10) tronconique ou évasée, .dont le diamètre va en se réduisant dans la direction distale, propre, dans le cas de l'implantation d'une valve (4) cardiaque, à prendre appui contre la paroi (102) du ventricule ou de l'oreillette correspondante du coeur.

19. Ensemble (1) selon la revendication 17 ou la revendication 18, **caractérisé en ce que** le stent (2) présente une portion supplémentaire d'appui (10) reliée par des tiges filiformes (13) à ladite portion distale (12) ou à ladite portion cylindrique (11), ces tiges filiformes (13) ayant des longueurs telles que cette portion supplémentaire d'appui (10) est placée au-delà des ostia coronaires.

20. Ensemble (1) selon l'une des revendications 1 à 19, **caractérisé en ce que** le stent (2) présente des crochets (15) mobiles entre une position de retrait, qu'ils occupent avant expansion du stent (2), et une position de déploiement, dans laquelle ils sont amenés lors du déploiement du stent (2) et dans laquelle ils sont insérés dans une paroi (103, 100) délimitant le conduit corporel.

21. Ensemble (1) selon l'une des revendications 1 à 20, **caractérisé en ce que** le stent (2) présente une portion (10) proche, en regard ou sur l'anneau valvulaire (103), ayant une force radiale élevée.

## Claims

1. A kit for placing a prosthetic valve (4) in a duct in the body, especially a heart valve, and in particular an aortic valve, comprising:
- the prosthetic valve (4) to be implanted;
- a radially expandable framework (2), called a stent, comprising at least one zone (10, 11, 14) intended to be expanded to allow this stent (2), in the expanded state, to bear against the wall of the body duct (100) to be fitted with the valve, this bearing making it possible to immobilize this stent (2) with respect to this wall; and
- means (25, 31; 50, 51) for mounting the valve (4) with respect to the stent (2), making it possible to connect the valve (4) to the stent (2) in such a way that the placement of the stent (2) allows the valve (4) to be mounted in the body duct (100);
expansion means such as a balloon catheter being provided to trigger expansion of the stent (2) at the implantation site;
which kit (1) is **characterized in that**:
- there is only one framework (2), the portion of this framework comprising said at least one zone (10, 11,14) intended to be expanded forming a unitary part with the portion of this framework receiving the valve or intended to receive the valve.
- the valve (4) and the stent (2) are designed in such a way that, at the moment when the stent (2) is expanded, the valve (4) is situated outside the zone or zones (10, 11, 14) of the stent (2) which are subjected to said expansion means.

2. The kit (1) as claimed in claim 1, **characterized in that** the stent (2) comprises a zone (12) for mounting of the valve (4), which zone (12) is distinct from the zone or zones (10, 11, 14) of the stent (2) to be expanded, and **in that** said mounting means (25, 31) connect the valve (4) to this mounting zone (12).

3. The kit (1) as claimed in claim 2, **characterized in that** said mounting means (25, 31) comprise conduits (31) formed in, or fixed on, the peripheral wall (30) of the valve (4), and arms (25) arranged on the stent (2), or vice versa, the peripheral wall (30) of the valve (4) being able to be engaged and slide on these arms (25).

4. The kit (1) as claimed in claim 1, **characterized in that** said mounting means (50, 51) are designed in such a way that the valve (4) is axially movable with respect to the stent (2) between a position of non-implantation , in which it is situated outside the zone or zones (10, 11, 14) of the stent (2) which are to be expanded, and a position of implantation, which it can reach after expansion of the stent (2) in the body duct (100), in which it is immobilized axially with respect to the stent (2).

5. The kit (1) as claimed in claim 4, **characterized in that** said mounting means (50, 51) comprise one or more of the following arrangements:
- fastening members such as spikes, hooks or claws which are mounted on the valve and are able to be inserted into the wall delimiting said body duct; these fastening members can be oriented radially with respect to the valve so as to be able to be inserted into said wall upon radial deployment of the valve, or they can be oriented tangentially with respect to the valve so as to be able to be inserted into said wall upon a pivoting of the valve about its axis or upon a longitudinal movement with respect to the stent;
- burstable vesicles which are filled with biological adhesive or other suitable adhesive product and are placed on the outer face of the valve, these vesicles being able to burst when the valve is brought into its position of implantation, in particular by their being crushed between the valve and the stent;
- at least one circular or helical wire or band integrated in the peripheral wall of the valve and having a shape memory, so that it keeps the valve pressed against the stent in the position of implantation of this valve;
- conduits (50) formed in, or fixed on, the peripheral wall of the valve, and rods formed on the stent, or vice versa, these rods (53) being able to be engaged and being able to slide through these conduits (50) as the valve (4) moves from its position of non-implantation to its position of implantation;
- means such as hooks in order to immobilize these conduits (50) with respect to these rods (53) in said position of implantation;
- wires (55) can be connected to the ends of said rods (53) and can pass through said conduits (50) in order to easily guide these rods (53) in these conduits (50).

6. The kit (1) as claimed in claim 1, **characterized in that** the means (60) for mounting the valve (4) with respect to the stent (2) are designed in such a way that, beyond a threshold of expansion of the stent, they permit a different expansion of the valve (4) and of the stent (2), so that a variation in the degree of expansion of the stent (2) has no effect on the degree of expansion of the valve (4).

7. The kit (1) as claimed in any of claims 1 to 6, **characterized in that** the stent (2) and/or the valve (4) comprise one or more elements (60) limiting the final diameter of the valve (4), in particular in the area of the commissure points of the valvules thereof.

8. The kit (1) as claimed in any of claims 1 to 7, **characterized in that** the valve (4) has a peripheral wall (30) with a diameter not constant in the axial direction, in particular a frusto-conical shape whose diameter decreases in the distal direction, and **in that** the zone (12) of the stent (2) intended to receive this peripheral wall (30) of the valve (4) has a shape corresponding to that of this peripheral wall (30).

9. The kit (1) as claimed in any of claims 1 to 8, **characterized in that** the stent (2) has a middle portion with a smaller diameter than its end portions, in particular in the form of two inverted truncated cones or an hourglass shape, the valve having a shape corresponding to that zone of the stent in whose area it is intended to be mounted.

10. The kit (1) as claimed in any of claims 1 to 9, **characterized in that**:
- the valve (4) has a peripheral wall (30);
- the stent (2) has, in the distal continuation of that zone (12) of the stent (2) intended to receive the valve (4), a foldable portion (45); this foldable portion (45) is movable between an extended position, in which it is situated in the distal continuation of said zone (12), and a folded position, in which it is placed against the inner face of the peripheral wall (30) of the valve (4) and traps this peripheral wall (30) between it and said zone (12) of the stent (2), and
- retaining means (46) are provided for keeping this foldable portion (45) in this folded position.

11. The kit (1) as claimed in claim 10, **characterized in that** said retaining means are formed by a wire (46) made of a material which is rigid but has a degree of elastic flexibility, for example a metal material having an undulated form and extending over the entire circumference of said foldable portion (45).

12. The kit (1) as claimed in any of claims 1 to 11, **characterized in that** the stent (2) comprises a sheath (3) made of an impermeable biocompatible material and at least partially covering it.

13. The kit (1) as claimed in claim 12, for placement of an aortic valve, **characterized in that** the sheath has lateral openings (61) which can be positioned opposite the coronary ostia (101) at the time of implantation.

14. The kit (1) as claimed in claim 12 or claim 13, **characterized in that**, in the case where it comprises said foldable portion (45), this foldable portion (45) is formed by a continuation of the sheath (3), forming a sleeve beyond that zone (12) of the stent (2) intended to receive the valve (4).

15. The kit (1) as claimed in any of claims 12 to 14, **characterized in that** the stent (2) has, fixed on said sheath (3), at least one inflatable peripheral chamber (41, 43) which can be inflated in order to form a seal ensuring leaktightness between the stent (2) and the wall of the body duct (100) to be fitted with the valve.

16. The kit (1) as claimed in claim 15, **characterized in that** the stent (2) has two inflatable peripheral chambers (41, 43) placed either side of that portion (11) of the stent (2) intended to bear against a cardiac valvular ring (103).

17. The kit (1) as claimed in any of claims 1 to 16, **characterized in that** the stent (2) has a cylindrical portion (11) which can bear against a cardiac valvular ring (103), and a distal portion (12) connected to this cylindrical portion (11).

18. The kit (1) as claimed in any of claims 1 to 17, **characterized in that** the stent (2) has a frustoconical or widened proximal portion (10) whose diameter decreases in the distal direction and able, in the case of implantation of a heart valve (4), to bear against the wall (102) of the ventricle or corresponding auricle of the heart.

19. The kit (1) as claimed in claim 17 or claim 18, **characterized in that** the stent (2) has a supplementary bearing portion (10) connected by filiform rods (13) to said distal portion (12) or to said cylindrical portion (11), these filiform rods (13) having lengths such that this supplementary bearing portion (10) is positioned beyond the coronary ostia.

20. The kit (1) as claimed in any of claims 1 to 19, **characterized in that** the stent (2) has hooks (15) which are movable between a retracted position, which they occupy before expansion of the stent (2), and a position of deployment into which they are brought upon deployment of the stent (2) and in which they are inserted into a wall (103, 100) delimiting the body duct.

21. The kit (1) as claimed in any of claims 1 to 20, **characterized in that** the stent (2) has a portion (10) near, opposite or on the valvular ring (103) and having a high radial force.

## Patentansprüche

1. Gruppe zum Einsetzen eines prothetischen Ventils (4) in eine Körperleitung, vor allem eines Herzventils und insbesondere eines Aortaventils, umfassend:
- das zu implantierende prothetische Ventil (4),
- ein Stent genanntes radial dehnbares Gestell (2), das mindestens einen Bereich (10, 11, 14) umfasst, der dazu bestimmt ist, gedehnt zu werden, um diesem Stent (2) im gedehnten Zustand zu erlauben, sich an der Wand der auszustattenden Körperleitung (100) abzustützen, wobei dieser Stent (2) durch diese Abstützung an dieser Wand immobilisiert wird, und
- Mittel (25, 31; 50, 51) zur Montage des Ventils (4) in Bezug zum Stent (2), die es erlauben, das Ventil (4) mit dem Stent (2) derart zu verbinden, dass die Platzierung des Stents (2) die Montage des Ventils (4) in der Körperleitung (100) ermöglicht,
Dehnmittel wie zum Beispiel ein Ballonkatheter, die dazu vorgesehen sind, die Ausdehnung des Stents (2) auf Ebene der Implantationsstelle hervorzurufen,
wobei die Gruppe (1) **dadurch gekennzeichnet ist, dass**:
- das Gestell (2) einzig ist, wobei der Teil dieses Gestells, der den mindestens den einen Bereich (10, 11, 14) umfasst, der dazu bestimmt ist, ausgedehnt zu werden, mit dem Teil dieses Gestells, das das Ventil aufnimmt oder zur Aufnahme des Ventils bestimmt ist, ein einziges Stück bildet, und
- das Ventil (4) und der Stent (2) derart ausgebildet sind, dass sich das Ventil (4) in dem Augenblick, in dem die Ausdehnung des Stents (2) durchgeführt wird, außerhalb des oder der Bereiche (10, 11, 14) des Stents (2) befindet, die den Dehnmitteln ausgesetzt sind.

2. Gruppe (1) nach Anspruch 1, **dadurch gekennzeichnet, dass** der Stent (2) einen Bereich (12) zur Montage des Ventils (4) umfasst, der sich von dem oder den auszudehnenden Bereichen (10, 11, 14) des Stents (2) unterscheidet, und **dadurch**, dass die Montagemittel (25, 31) das Ventil (4) mit diesem Montagebereich (12) verbinden.

3. Gruppe (1) nach Anspruch 2, **dadurch gekennzeichnet, dass** die Montagemittel (25, 31) Leitungen (31) umfassen, die in der peripheren Wand (30) des Ventils (4) eingearbeitet sind oder auf ihr befestigt, und Arme (25), die auf dem Stent (2) angebracht sind, oder umgekehrt, wobei die periphere Wand (30) des Ventils (4) einführbar ist und imstande ist, auf diesen Armen (25) zu gleiten.

4. Gruppe (1) nach Anspruch 1, **dadurch gekennzeichnet, dass** die Montagemittel (50, 51) derart ausgebildet sind, dass das Ventil (4) in Bezug zum Stent (2) zwischen einer nicht implantierten Stellung, in der es sich außerhalb des oder der Bereiche (10, 11, 14) des Stents (2), die auszudehnen sind, befindet, und einer Implantationsstellung, die es nach Ausdehnung des (2) in der Körperleitung (100) imstande ist einzunehmen, in der es axial in Bezug zum Stent (2) immobilisiert ist, axial bewegbar ist.

5. Gruppe (1) nach Anspruch 4, **dadurch gekennzeichnet, dass** die Montagemittel (50, 51) eine oder mehrere der folgenden Anordnungen umfassen:
- Befestigungsorgane wie zum Beispiel Spitzen, Haken oder Krallen, die auf dem Ventil angebracht sind und dazu geeignet, in die Wand eingefügt zu werden, die die Körperleitung begrenzt, wobei diese Befestigungsorgane imstande sind, radial in Bezug zum Ventil ausgerichtet zu werden, um imstande zu sein, bei der radialen Ausdehnung des Ventils in die Wand eingefügt zu werden, oder imstande sind, tangential in Bezug zum Ventil ausgerichtet zu werden, so dass sie imstande sind, bei einem Drehen des Ventils um seine Achse oder bei einer Längsbewegung in Bezug zum Stent in die Wand eingefügt zu werden,
- aufbrechbare, mit biologischem Klebstoff oder einen anderen geeigneten Haftprodukt gefüllte Blasen, die auf der Außenseite des Ventils platziert sind, wobei diese Blasen dazu geeignet sind aufzuplatzen, wenn das Ventil an seine Implantationsstelle geführt ist, vor allem durch Zerdrücken zwischen dem Ventil und dem Stent,
- mindestens ein Band oder einen kreisrunden oder spiralförmigen Draht, der in der peripheren Wand des Ventils integriert ist, mit einem Formengedächtnis, das derart ist, dass es/er das Ventil in den der Implantationsstellung dieses Ventils gegen den Stent gedrückt hält,
- Leitungen (50), die in der peripheren Wand des Ventils eingearbeitet sind oder auf ihr befestigt und Stifte, die auf dem Stent angebracht sind, oder umgekehrt, wobei diese Stifte (53) imstande sind, beim Übergang des Ventils (4) aus seiner nicht implantierten Stellung in seine Implantationsstellung in diese Leitungen (50) eingeführt zu werden und durch sie zu gleiten,
- Mittel wie zum Beispiel Haken imstande sind, die Leitungen (50) in Bezug zu diesen Stiften (53) in der Implantationsstellung zu immobilisieren,
- Drähte (55) imstande sind, mit den Enden der Stifte (53) verbunden zu werden und durch die Leitungen (50) durchzugehen, um diese Stifte (53) in diesen Leitungen (50) problemlos zu führen.

6. Gruppe (1) nach Anspruch 1, **dadurch gekennzeichnet, dass** die Mittel (60) zur Monate des Ventils (4) in Bezug zum Stent (2) derart ausgebildet sind, um jenseits einer Ausdehnungsschwelle des Stents eine unterschiedliche Ausdehnung des Ventils (4) und des Stents (2) zu erlauben, so dass eine Schwankung im Ausdehnungsgrad des Stents (2) keine Auswirkung auf den Ausdehnungsgrad des Ventils (4) hat.

7. Gruppe (1) nach einem der Ansprüche 1 bis 6, **dadurch gekennzeichnet, dass** der Stent (2) und/oder das Ventil (4) ein oder mehrere Elemente (60) umfassen, die den Enddurchmesser des Ventils (4) vor allem auf Ebene der Winkelpunkte der Klappen desselben begrenzen.

8. Gruppe (1) nach einem der Ansprüche 1 bis 7, **dadurch gekennzeichnet, dass** das Ventil (4) eine periphere Wand (30) mit einem Durchmesser aufweist, der in axialer Richtung nicht konstant ist, vor allem eine kegelstumpfförmige Form, deren Durchmesser sich in die distale Richtung reduziert, und **dadurch**, dass der Bereich (12) des Stents (2), der zur Aufnahme dieser peripheren Wand (30) des Ventils (4) bestimmt ist, eine Form aufweist, die der dieser peripheren Wand (30) entspricht.

9. Gruppe (1) nach einem der Ansprüche 1 bis 8, **dadurch gekennzeichnet, dass** der Stent (2) einen mittleren Abschnitt mit einem kleineren Durchmesser als seine Endabschnitte aufweist, vor allem als zwei umgekehrte Kegelstümpfe oder Doppelkegel, wobei das Ventil eine Form aufweist, die dem Bereich des Stents entspricht, auf dessen Ebene es zur Montage bestimmt ist.

10. Gruppe (1) nach einem der Ansprüche 1 bis 9, **dadurch gekennzeichnet, dass**:
- das Ventil (4) eine periphere Wand (30) aufweist,
- der Stent (2) distal in Verlängerung des Bereichs (12) des Stents (2), der zur Aufnahme des Ventils (4) bestimmt ist, einen klappbaren Abschnitt (45) aufweist, wobei dieser klappbare Abschnitt (45) zwischen einer ausgestreckten Stellung, in der er sich in Verlängerung des Bereichs (12) der distalen Seite befindet, und einer geklappten Stellung, in der er gegen die Innenseite der peripheren Wand (30) des Ventils (4) platziert ist, bewegbar ist und diese periphere Wand (30) zwischen ihr und dem Bereich (12) des Stents (2) einschließt, und
- Haltemittel (46) vorgesehen sind, um diesen klappbaren Abschnitt (45) in dieser geklappten Stellung zu halten.

11. Gruppe (1) nach Anspruch 10, **dadurch gekennzeichnet, dass** die Haltemittel von einem Draht (46) aus einem starren Material gebildet werden, das jedoch einen elastischen Biegsamkeitsgrad aufweist, wie zum Beispiel ein metallisches Material mit einer welligen Form und sich über den gesamten Umfang des klappbaren Abschnitts (45) erstreckt.

12. Gruppe (1) nach einem der Ansprüche 1 bis 11, **dadurch gekennzeichnet, dass** der Stent (2) eine Hülle (3) aus einem wasserundurchlässigen biologisch kompatiblen Material umfasst, das ihn mindestens teilweise bedeckt.

13. Gruppe (1) nach Anspruch 12 zum Einsetzen eines Aortaventils, **dadurch gekennzeichnet, dass** die Hülle seitliche Öffnungen (61) aufweist, die geeignet sind, im Augenblick der Implantation gegenüber den Koronarostien (101) zu gelangen.

14. Gruppe (1) nach Anspruch 12 oder Anspruch 13, **dadurch gekennzeichnet, dass**, wenn sie den klappbaren Abschnitt (45) umfasst, dieser klappbare Abschnitt (45) von einer Verlängerung der Hülle (3) gebildet wird, die jenseits des Bereichs (12) des Stents (2), der zur Aufnahme des Ventils (4) bestimmt ist, eine Muffe bildet.

15. Gruppe (1) nach einem der Ansprüche 12 bis 14, **dadurch gekennzeichnet, dass** der Stent (2) mindestens eine auf der Hülle (3) befestigte aufblasbare periphere Kammer (41, 43) aufweist, die derart aufblasbar ist, dass sie eine Dichtung bildet, die die Dichtigkeit zwischen dem Stent (2) und der Wand der auszustattenden Körperleitung (100) bildet.

16. Gruppe (1) nach Anspruch 15, **dadurch gekennzeichnet, dass** der Stent (2) zwei aufblasbare periphere Kammern (41, 43) aufweist, die auf einer und der anderen Seite des Abschnitts (11) des Stents (2) platziert sind, der dazu bestimmt ist, sich auf einem Herzkappenring (103) abzustützen.

17. Gruppe (1) nach einem der Ansprüche 1 bis 16, **dadurch gekennzeichnet, dass** der Stent (2) einen zylindrischen Abschnitt (11) aufweist, der dazu geeignet ist, sich auf einem Herzklappenring (103) abzustützen, und einen distalen Abschnitt (12), der mit diesem zylindrischen Abschnitt (11) verbunden ist.

18. Gruppe (1) nach einem der Ansprüche 1 bis 17, **dadurch gekennzeichnet, dass** der Stent (2) einen proximalen kegelstumpfförmigen oder erweiterten Abschnitt (10) aufweist, dessen Durchmesser sich in die distale Richtung verringert, der im Fall der Implantation eines Herzventils (4) dazu geeignet ist, sich auf der Wand (102) der Kammer oder des entsprechenden Vorhofs des Herzens abzustützen.

19. Gruppe (1) nach Anspruch 17 oder Anspruch 18, **dadurch gekennzeichnet, dass** der Stent (2) einen zusätzlichen Abstützabschnitt (10) aufweist, der durch dünne Stifte (13) mit dem distalen Abschnitt (12) oder dem zylindrischen Abschnitt (11) verbunden ist, wobei diese dünnen Stifte (13) Längen haben, die derart sind, dass dieser zusätzliche Abstützabschnitt (10) jenseits der Koronarostien platziert ist.

20. Gruppe (1) nach einem der Ansprüche 1 bis 19, **dadurch gekennzeichnet, dass** der Stent (2) Haken (15) aufweist, die zwischen einer zurückgezogenen Stellung, die sie vor der Ausdehnung des Stents (2) einnehmen, und einer entfalteten Stellung, in welche sie bei Entfaltung des Stent (2) geführt werden und in der sie in eine Wand (103, 100) eingeführt werden, die die Körperleitung begrenzt, bewegbar sind.

21. Gruppe (1) nach einem der Ansprüche 1 bis 20, **dadurch gekennzeichnet, dass** der Stent (2) einen nahen Abschnitt (10) gegenüber oder auf dem Klappenring (103) aufweist, der eine erhöhte Radialkraft hat.
